# EUROPEAN PATENT APPLICATION

(11) **EP 2 915 878 A1**
(43) Date of publication of application: **09.09.2015**
(21) Application number: 14158437.5
(22) Date of filing: 07.03.2014
(51) Int. Cl.: C12N 5/0787

(54) **Method and device for conserving viable and functional human polymorphonuclear neutrophils**

(71) Applicant: INSTITUT PASTEUR, 75015 Paris (FR)
(72) Inventor: Marteyn, Benoit, 75005 Paris (FR); Sansonetti, Philippe, 75014 Paris (FR); Monceaux, Valérie, 78310 Maurepas (FR); Ungeheuer, Marie-Noelle, 78310 Maurepas (FR)
(74) Representative: Marcadé, Véronique

(57) **Abstract**

A method for keeping leukocytes alive *ex vivo* or *in vitro,* comprising maintaining the leukocytes in a medium comprising from 3 to 10 mM of glucose, in hypoxic conditions with P(O₂) ≤ 10 mM Hg.

## Description

The present invention pertains to the fields of transfusion and of cell culture. More particularly, the invention relates to new storage conditions for leukocytes, in particular for polymorphonuclear neutrophils (neutrophils), which allow an increased survival of functional cells, as well as to a method for storing said leukocytes and a device for storing and transporting them.

Polymorphonuclear neutrophils are the most abundant circulating white blood cell population (70% in human). However, human neutrophils are certainly the most difficult immune cells to study *in vitro,* regarding their short lifespan under atmospheric conditions (pO₂ atm. = 160 mmHg), which does not exceed 8 hours. Neutrophils survival and cell death mechanisms are tightly regulated regarding their anti-infectious function and successful inflammation resolution respectively¹. Apoptosis is the main cell death pathway² and several anti-apoptotic factors (*eg.*,. G-CSF³, GM-CSF⁴) were shown to extend purified neutrophil longevity.

Increasing purified neutrophils survival represents a major challenge to promote both their study *in vitro* and their clinical use, especially for neutrophil transfusion, which efficiency is still discussed, as rapid neutrophil apoptosis may be responsible for its failure⁵. Several strategies have been evaluated for their potential beneficial effect on neutrophil survival, including the evaluation of optimal anticoagulant molecules⁶.

To the aim of increasing purified neutrophils survival, the inventors inspired of and mimicked physiological conditions encountered by neutrophils in their niche during the maturation process. Neutrophils maturation occurs in the bone marrow from hematopoietic stem cells differentiating from myeloblasts to mature neutrophils⁷. These events occur in an hypoxic environment in which maturating neutrophils remain 3-5 days⁸. In this context, maturating cells produce energy required for their development through glycolysis rather than respiration in an HIF-1-dependent manner⁹. HIF-1 is an heterodimeric (α-β) transcriptional regulator; its α subunit abundance is regulated upon hydroxylation by prolyl-hydroxylases (PHD1, PHD2 and PHD3) under normoxic conditions^{10,11}, followed by ubiquitination and proteasomal degradation¹². Following their production, mature neutrophils remain within the bone marrow for 4-6 additional days¹³.

Once released into the blood circulation through sinusoids, neutrophils encounter oxygenated environments. Sinusoidal oxygen pressure is not precisely known *in situ,* however it is estimated between artery (pO₂ a. = 75-100 mmHg) and venous (pO₂ v. = 30-50 mmHg) oxygen pressures¹⁴. In this oxygenated context, human neutrophils lifespan remains discussed as varying among studies from 10 h to 90 h^{13,15,16,17} before clearance occurring equally in the bone marrow, the liver and the spleen¹⁸. A low oxygen environment is favourable to neutrophil lifespan, which is mediated by HIF-1α¹². Inhibition of PHD3 by addition of Dimethyloxalylglycine (DMOG), a pan-hydroxylase inhibitor, decreases apoptosis under normoxic conditions¹⁹. Consistently, containing few mitochondria, neutrophils rely mainly on anaerobic glycolysis for energy production^{20,21}.

As disclosed in the experimental part below, the inventors now demonstrated a synergistic effect of anoxia, glycolysis induction and HIF stabilization on neutrophils survival. Optimum storage conditions are disclosed, which, following collection and purification of neutrophils with citrate, allow survival rates of 74±2% after 20 h and 49±3% after 48 h storage in an appropriate culture medium, and even more after storage in plasma under anoxic conditions; moreover, the viable neutrophils remain functional under these conditions. Preserving neutrophils in such an environment allowed for the first to perform plasmid transfection and siRNA gene silencing on human purified neutrophils, which opens new perspectives for neutrophil cell biology and physiopathological studies.

A first aspect of the present invention is a method for keeping leukocytes alive *ex vivo* or *in vitro,* comprising maintaining the leukocytes in an appropriate medium comprising from 3 to 10 mM of glucose, preferably from 3 to 6 mm mM of glucose, in hypoxic conditions with P(O₂) ≤ 10 mM Hg.

According to a preferred embodiment of the method, the partial pressure of dioxygen is inferior or equal to 1 mM Hg (P(O₂) ≤ 1 mM Hg), which will be considered here after as "anoxic conditions". To this aim, the leukocytes are preferably obtained from a donor by a process preventing their contact with oxygen, for example through apheresis performed in conditions such that the cells are not contacted by O₂. They are then kept in anaerobic conditions, for example in a closed (and preferably gas-free) oxygen-tight device, or in an anaerobic incubator.

As used herein, an "oxygen-tight", or "oxigen- impermeable" device is a storage container or storage container system that is impermeable to oxygen. In accordance with the present invention, an oxygen-impermeable storage container is a container, pouch, bag, or bottle that is constructed of a material compatible with a biological fluid, such as whole blood or a blood component and is preferably capable of withstanding centrifugation and sterilization. Such containers are known in the art and include, *e.g.,* blood collection and satellite bags. Storage containers of use in the instant method can be made of plasticized polyvinyl chloride, *e.g.,* PVC plasticized with dioctylphthalate, diethylhexylphthalate, or trioetyltrimellitate. The bags may also be formed from polyolefin, polyurethane, polyester, and polycarbonate. In one embodiment, the storage container itself is constructed of an oxygen--impermeable material. Impermeable materials are routinely used in the art and any suitable material can be used. Existing systems use oxygen-impermeable receptacles composed of layers of ethylene vinyl alcohol copolymer and modified ethylene vinyl acetate copolymer, impermeable to oxygen ingress. In another embodiment, the leukocytes are transferred into a first storage container which is a component of a storage container system that is impermeable to oxygen. Such systems include, but are not limited to, use of an oxygen-impermeable over wrap or over bag which encloses the storage container.

In what precedes, an "appropriate medium" can be either plasma or any culture medium appropriate for leukocytes survival. Examples of culture media appropriate for performing the method according to the invention include:
- RMPI 1640 + 10 mM Hepes + 10% FBS,
- RMPI 1640 with L-Glu + 25 mM Hepes + 10% FBS,
- IMDM + 10% autologous serum, etc.

Of course, in the above media, the fetal bovine serum (FBS) can be replaced by newborn calf serum (NBS) or by inactivated human serum.

According to a particular embodiment of the invention, the culture medium is a synthetic medium which has been treated for removing dioxygen therefrom prior to transferring the leukocytes into it. This can be done by any technique known by the skilled artisan, such as gazing the medium with an oxygen-free gaz (for example CO₂/N₂ or CO₂/H₂/N₂).

In case the medium is a culture medium, it will preferably comprise an effective amount of a compound that stabilizes the hypoxia inducible factor-1 (HIF-1).

Of course, the compound used to stabilize HIF-1 can do so either by a direct action of one of HIF-1 subunits (in particular, HIF-1α), or by an indirect action on HIF-1. For example, since the HIF-1α subunit is regulated by hydroxylation, in particular by the oxygen-sensitive propyl hydroxylase-3 (PHD-3) and by transcriptional inactivation following asparaginyl hydroxylation by factor inhibiting HIF (FIH), a compound that inhibits these, or that inhibits the 2-oxoglutarate dioxygenase activity of any other enzyme implicated in HIF stability, is also considered as a "compound that stabilizes HIF-1", according to the present invention.

According to a preferred embodiment, the compound that stabilizes the hypoxia inducible factor-1 (HIF-1) comprises or consists of a prolyl hydroxylase inhibitor.

Due to safety and regulatory constraints, when the leukocytes are stored in view of a transfusion, the HIF-1-stabilizing compound must be appropriate for injection to a patient.

Non-limitative examples of compounds which can be used to stabilize HIF-1 when performing the above method include dimethyloxalylglycine (DMOG), the cyanoisoquinoline compounds disclosed in WO 2007/090068, the compounds capable of inhibiting HIF hydroxylase enzyme activity disclosed in WO 2012/106472, the compounds disclosed in US 2013/310565 and those disclosed in US 2013/245037.

According to a preferred embodiment, the compound that stabilizes HIF-1α is the dimethyloxalylglycine (DMOG). In this case, DMOG is preferably present in the medium at a concentration ranging from 8 to 50 µg/mL, more preferably from 25 to 40 µg/mL, for example in a concentration of about 32 µg/mL.

According to another preferred embodiment of the method according to the invention, the leukocytes are maintained in plasma, under hypoxic (P(O₂) ≤ 10 mM Hg) or anoxic conditions. If necessary, glucose is added to the plasma so that the final glucose concentration is between 3 and 10 mM, preferably between 3 and 6 mM. According to this embodiment, the plasma is preferably from the same donor as the leukocytes.

The present invention is particularly useful for maintaining leukocytes which are known to have a short lifespan *in vitro,* such as human granulocytes, and in particular polymorphonuclear neutrophils. Hence, according to a preferred embodiment of the present invention, the leukocytes comprise granulocytes. According to another preferred embodiment of the present invention, the leukocytes consist of granulocytes.

Granulocytes include polymorphonuclear neutrophils, basophils and eosinophils. According to a preferred embodiment of the present invention, the leukocytes comprise polymorphonuclear neutrophils. According to another preferred embodiment of the present invention, the leukocytes consist of polymorphonuclear neutrophils. In this case, the polymorphonuclear neutrophils are preferentially purified.

According to a preferred embodiment of the invention, illustrated in the experimental part below, more than 70% of the polymorphonuclear neutrophils remain viable after 20 hours storage. According to another embodiment of the invention, more than 80%, and even 90% of the polymorphonuclear neutrophils remain viable after 20 hours storage.

According to a preferred embodiment of the invention, illustrated in the experimental part below, more than 45% of the polymorphonuclear neutrophils remain viable after 48 hours storage.

According to a preferred embodiment of the invention, illustrated in the experimental part below, the viable polymorphonuclear neutrophils remain functional.

According to a particular embodiment of the present invention, the leukocytes comprise basophils. According to another particular embodiment of the present invention, the leukocytes consist of basophils.

According to a particular embodiment of the present invention, the leukocytes comprise eosinophils. According to another particular embodiment of the present invention, the leukocytes consist of eosinophils.

According to a particular embodiment of the present invention, the leukocytes comprise peripheral blood mononuclear cells (PBMCs). According to another particular embodiment of the present invention, the leukocytes consist of PBMCs.

According to a particular embodiment of the present invention, the leukocytes comprise monocytes. According to another particular embodiment of the present invention, the leukocytes consist of monocytes.

According to a particular embodiment of the present invention, the leukocytes comprise lymphocytes. According to another particular embodiment of the present invention, the leukocytes consist of lymphocytes.

According to a particular embodiment of the present invention, the leukocytes comprise T lymphocytes (LT). According to another particular embodiment of the present invention, the leukocytes consist of LT.

According to a particular embodiment of the present invention, the leukocytes comprise B lymphocytes (LB). According to another particular embodiment of the present invention, the leukocytes consist of LB.

According to a particular embodiment of the present invention, the leukocytes comprise macrophages. According to another particular embodiment of the present invention, the leukocytes consist of macrophages.

Several anticoagulant molecules are available for treating blood samples, including heparin, citrate, ACD (acid citrate dextrose or anticoagulant citrate-dextrose) and EDTA. Heparin and citrate are the most commonly used anticoagulants for clinical analysis or *in vitro* basic research. The inventors have tested the impact of the anticoagulant molecule used during blood collection on the survival of neutrophils subsequently purified from the blood samples, and noted that neutrophils survival rates 20 h- and 48 h-post purification were improved when blood was collected in the presence of citrate as compared to heparin (Example 2 below). They obtained even better results by using EDTA instead of citrate at blood collection. Hence, according to preferred embodiments of the present invention, the leukocytes have been purified from citrated venous blood or from blood treated with EDTA. According to preferred embodiments of the present invention, polymorphonuclear neutrophils have been purified from citrated venous blood or from blood treated with EDTA.

According to another aspect, the present invention pertains to an oxygen-tight container containing live leukocytes in a medium with P(O₂) ≤ 10 mM Hg and from 3 to 10 mM of glucose. Examples of containers include bags, bottles *etc.* Oxygen-impermeable materials are routinely used in the art and any suitable material can be used, such as, for example, plasticized polyvinyl chloride, *e.g.,* PVC plasticized with dioctylphthalate, diethylhexylphthalate, trioetyltrimellitate, polyolefin, polyurethane, polyester, and polycarbonate. According to a preferred embodiment, the volume of the container is comprised between 5 and 300 mL. According to a preferred embodiment, the medium is plasma or essentially plasma, possibly complemented with glucose.

According to another aspect, the present invention pertains to a device for transporting leukocytes, comprising an oxygen-tight container containing a culture medium as described above, and means appropriate for introducing cells into the culture medium. According to this embodiment, the oxygen-impermeable container is as described above and the means for introducing cells into the culture medium are preferentially designed for avoiding an important contact between the culture medium and air during the transfer of the cells into the device. Examples of such means include caps having a small diameter, for example a diameter of less than 5 mm, as well as silicone, rubber or plastic plugs which can be pierced by a needle, *etc.*

As recalled above, the leukocytes are preferably obtained by a process preventing their contact with oxygen, for example through apheresis performed in conditions such that the cells are not contacted by O₂, such as a closed device entirely made of oxygen-tight materials, including the tubes *etc.*

According to a preferred embodiment of devices according to the invention, the leukocytes comprise neutrophils. According to another embodiment, the leucocytes are neutrophils.

According to another aspect, the present invention concerns a culture medium for neutrophils, characterized in that it comprises between 3 and 6 mM of glucose and between 25 and 40 µg/mL of DMOG.

The present invention also pertains to a method for transfecting polymorphonuclear neutrophils, comprising (i) a step of pre-conditioning said polymorphonuclear neutrophils by transferring them into a medium as described above, or by maintaining them in plasma, (ii) a step of incubating them in hypoxic or anoxic conditions, and (iii) a transfection step.

A population of polymorphonuclear neutrophils which has been transfected according to the above method is also part of the present invention. In a preferred embodiment, at least 30% of these cells have been effectively transfected.

The invention is further illustrated by the following figure and examples.

### FIGURE LEGENDS

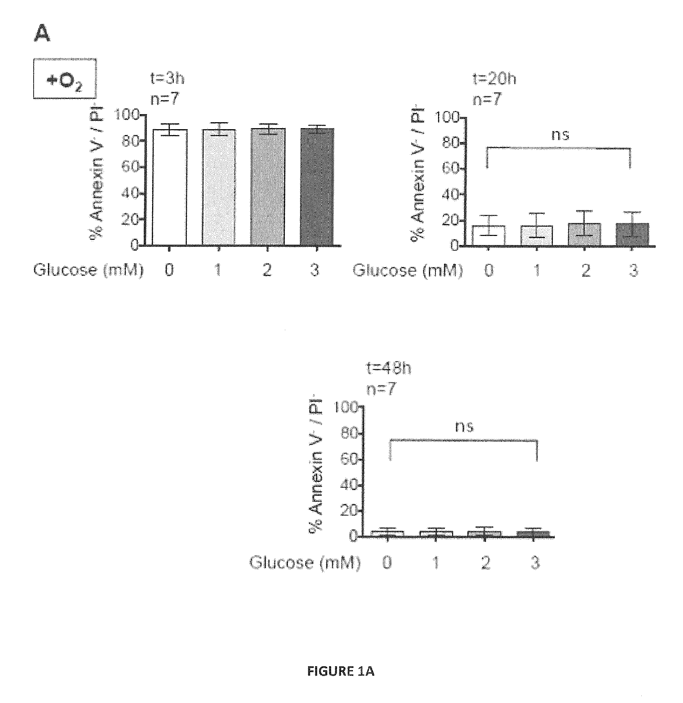

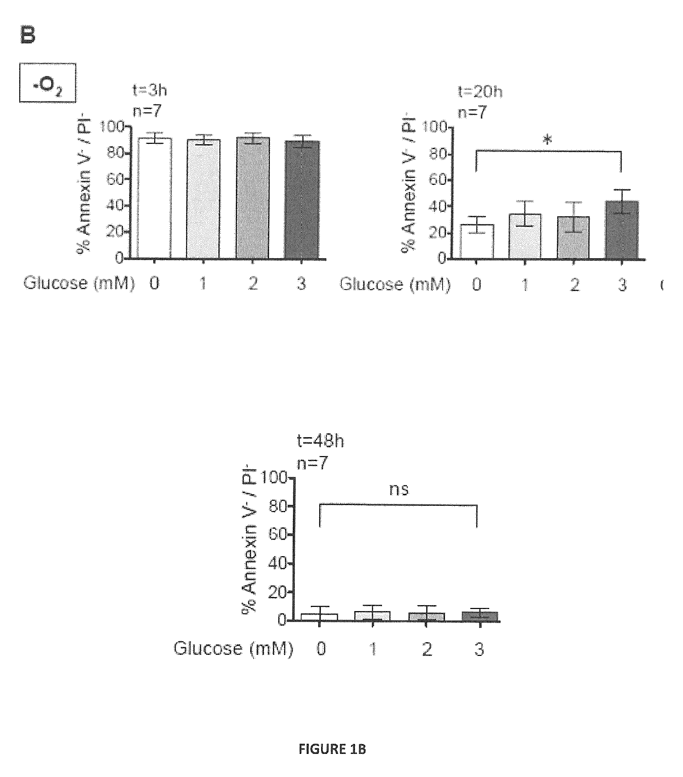

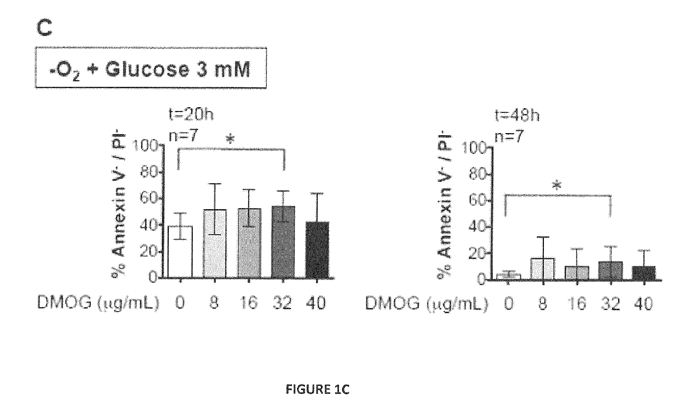

Figure 1. Optimization of human neutrophil survival conditions in the presence and absence of oxygen, with heparin as an anticoagulant. (A-B) Human neutrophils were purified on heparin-coated collection tubes (Method 1). Neutrophil survival was quantified by flow cytometry (Annexin V-/PI-) either under atmospheric conditions (20% O₂; (A)) or in the absence of oxygen (0% O₂; (B)). Purified neutrophil survival was determined after 3, 20 and 48 h storage in the presence of 0, 1, 2 or 3 mM glucose as indicated. Error bars indicate SD. 'ns' indicates P > 0.05, * indicates P < 0.05. Data represent n=7 individual blood samples, which were collected and analysed independently. (C) Neutrophil survival was quantified as described in (A) in the absence of oxygen (-O₂) with 3mM glucose and in the presence of 0, 8, 16, 32 or 40 µg/mL DMOG as indicated, after 20 or 48 h storage. Error bars indicate SD. * indicates P < 0.05. Data represent n=7 individual blood samples, which were collected and analysed independently.

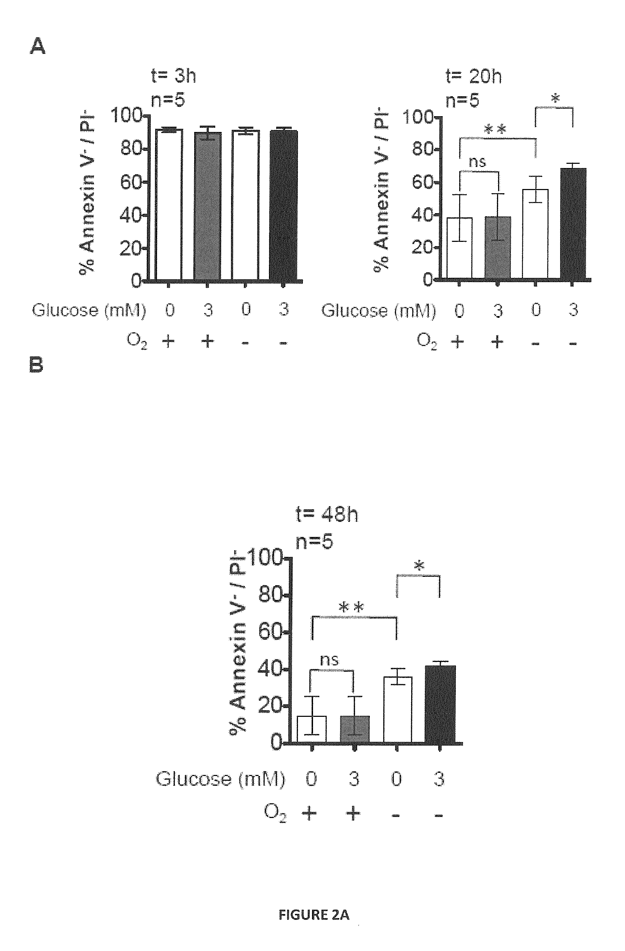

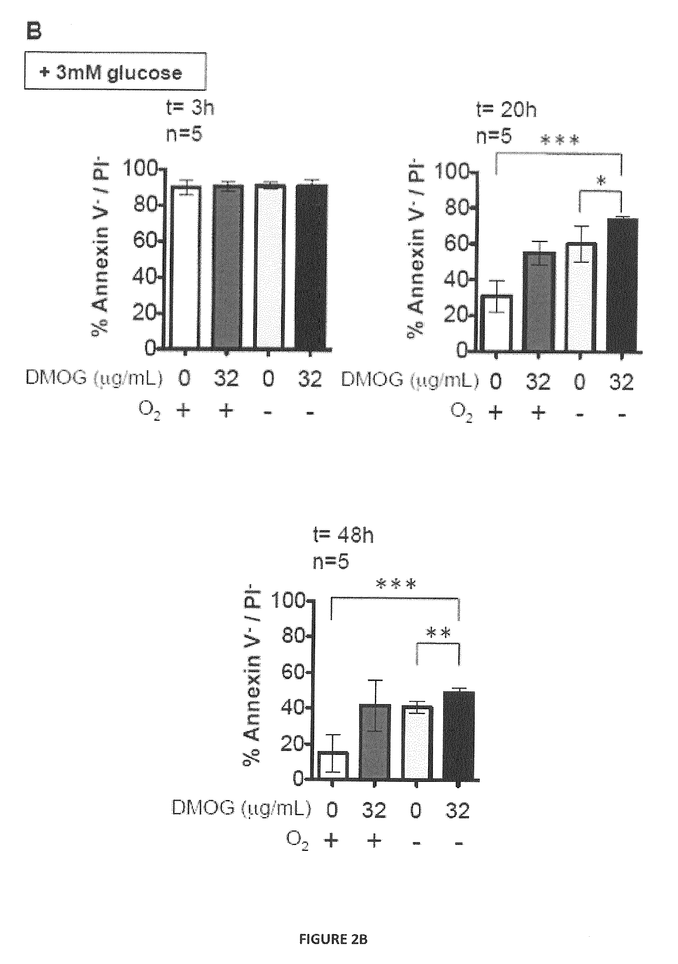

**Figure 2****. Optimization of human neutrophil survival conditions, with citrate as an anticoagulant.** (A) Human neutrophils were purified in the presence of citrate (Method 2). Neutrophil survival was quantified by flow cytometry (Annexin V-/PI-) either under atmospheric conditions (20% O₂) or in the absence of oxygen (0% O₂); with or without 3 mM glucose. Purified neutrophil survival was determined after 3, 20 and 48 h storage in the presence of 0, 1, 2 or 3 mM glucose as indicated. Error bars indicate SD. * indicates P < 0.05, ** indicates P < 0.01. Data represent n=5 individual blood samples, which were collected and analysed independently. (B) Neutrophil survival was quantified as described in (A) in the presence (+O₂) or absence of oxygen (-O₂) with 3mM glucose and in the presence or absence of 32 µg/mL DMOG as indicated, after 3, 20 or 48 h storage. Error bars indicate SD. * indicates P < 0.05, ** indicates P < 0.01, *** indicates P < 0.001. Data represent n=5 individual blood samples, which were collected and analysed independently.

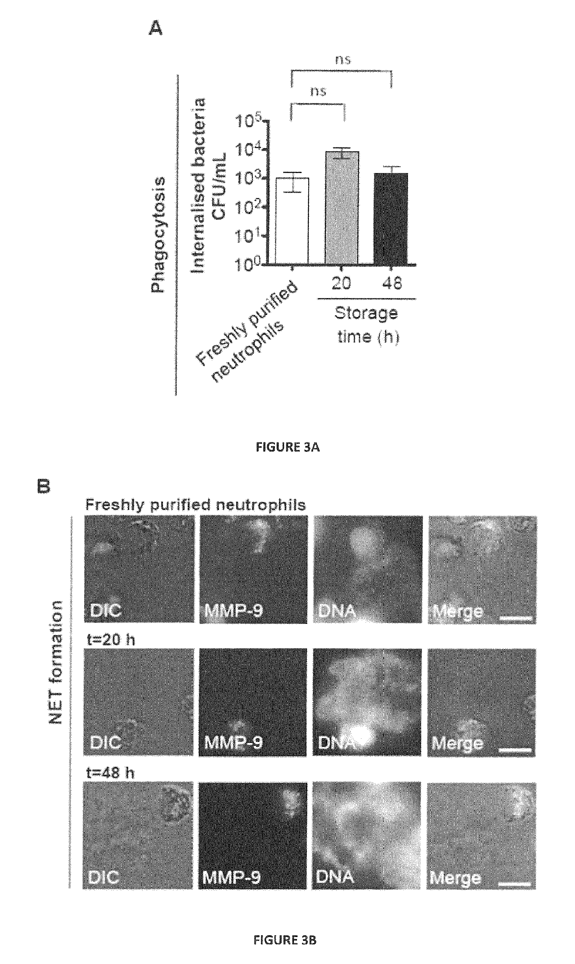

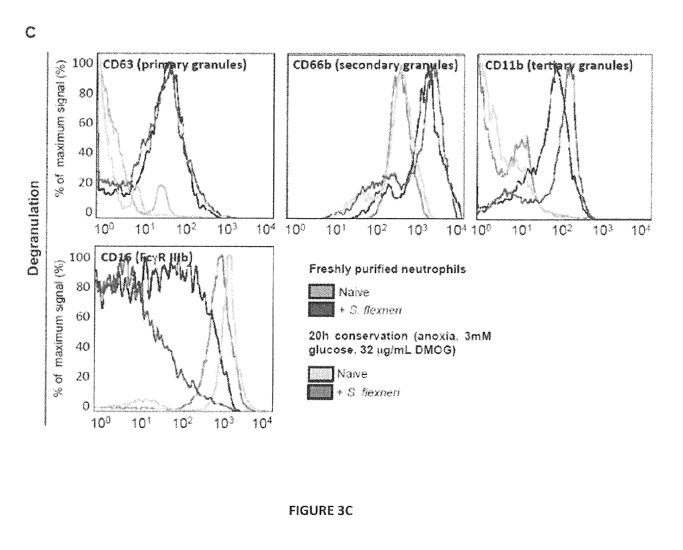

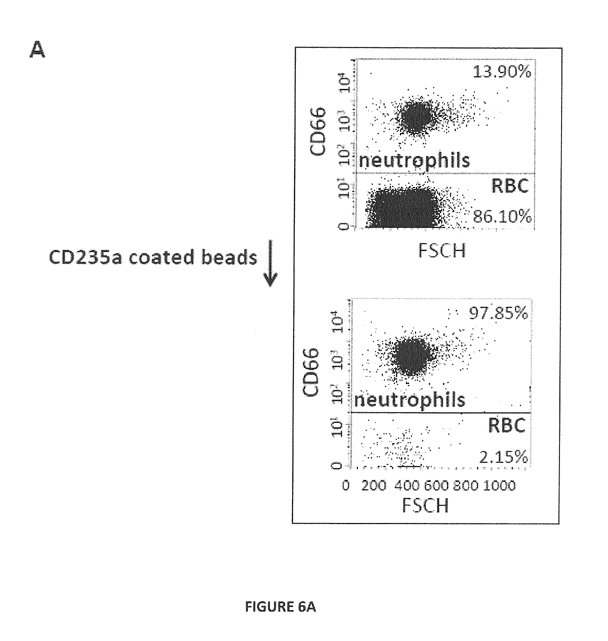

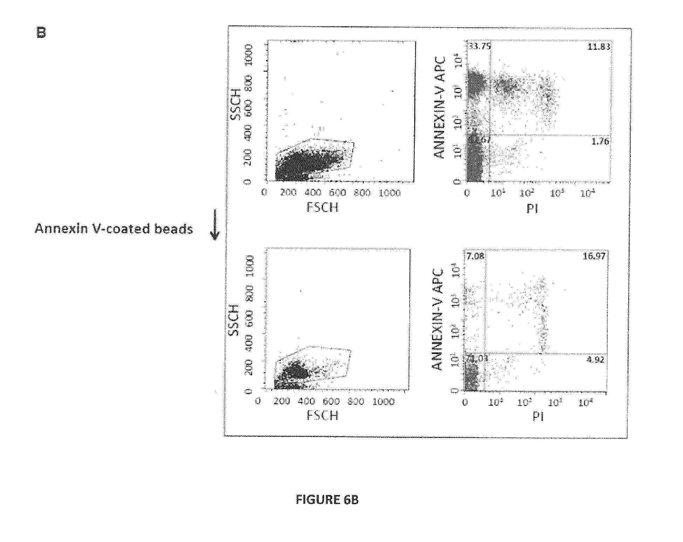

**Figure 3****. Stored neutrophils remain functional as compared to freshly purified neutrophils.** Neutrophils stored under anoxic conditions with 3mM glucose and 32 mg/mL DMOG were sorted on Annexin V-coated beads (Figure 6). Their phagocytosis (A), NET formation (B) and degranulation (C) properties were compared to freshly purified neutrophils. (A) *S*. *flexneri* phagocytosis assay was performed with either freshly purified or stored (20, 48 h) and viable neutrophils, at MOI 20 during 15 min at 37°C. Results are averaged from three independent experiments (n=3) performed in triplicate. Error bars indicate SD. 'ns' indicates P > 0.05. (B) Neutrophil NET formation was induced from either freshly purified or stored (20, 48 h) and viable neutrophils, in the presence of 25 nM PMA for 3 hours at 37°C. Immunofluorescence staining was performed with α-MMP-9 (green). DNA was stained with DAPI (blue). Results are representative of three independent experiments. Bars are 20 µm.(C) Neutrophil degranulation was assessed by flow cytometry detecting specific primary (CD63), secondary (CD66b), tertiary (CD11b) granules or CD16 (FcγR IIb). Freshly purified or stored (20, 48 h) and viable neutrophils (naive) were activated in the presence of *S. flexneri* (MOI 20). Results are representative of three independent experiments.

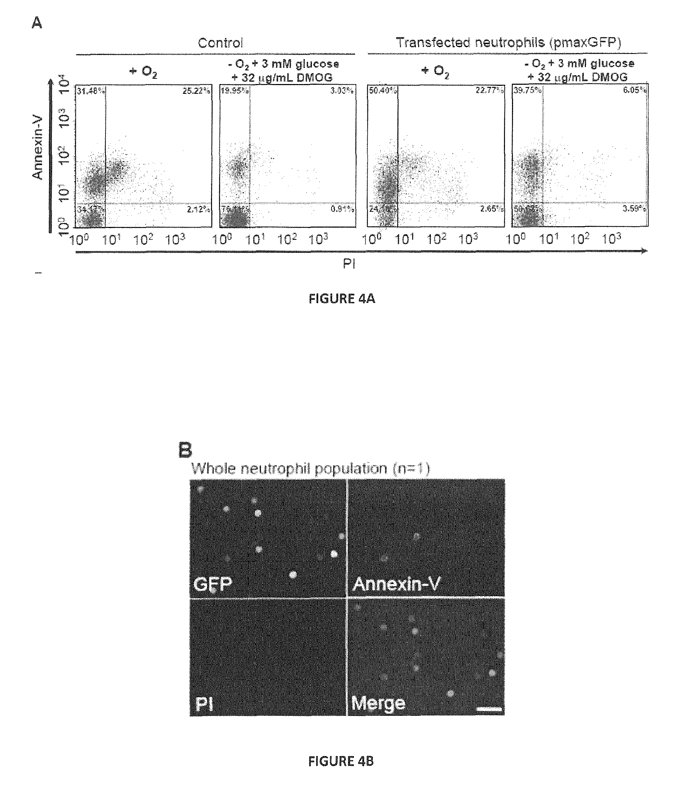

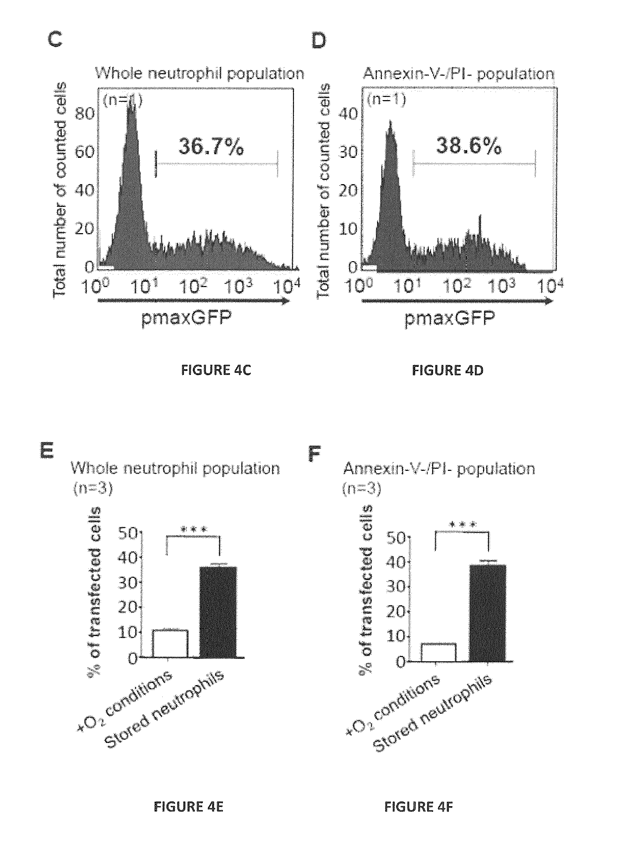

**Figure 4****. Neutrophil plasmid nucleofection optimization upon storage.** Neutrophil transfection efficiency by nucleofection was evaluated in the presence of oxygen (+O₂, 20%) or under anoxic conditions (-O₂, 0%) with 3 mM glucose and 32 mg/mL DMOG. Neutrophils purified on citrate were pre-conditioned in storage media during 2 h prior nucleofection. Using Y-01 program, neutrophils (2.10⁶ cells) were transfected with 2 µg pmaxGFP (Amaxa biosystems) with Nucleofector (Lonza). After 20 h transfection and cell recovery, neutrophils viability (A) and transfection efficiency (B-E) were analysed by flow cytometry. (A) Cell viability (Annexin V-/PI-) after nucleofection was compared to non-transfected cells (Control) in the presence of oxygen (+O₂) or upon storage (-O₂, + 3mM glucose + 32 µg/mL DMOG). Results are representative of three independent experiments. (B) Confocal imaging of whole transfected neutrophil population, detecting GFP (green), Annexin V (Red) and DNA (PI, blue) positive cells. Bar is 60 µm. Result is representative of three independent experiments. (C-D) Flow cytometry quantification of the GFP+ neutrophil proportion within the whole neutrophil population (C) or within viable (Annexin V-/PI-) population (D). Results are representative of three independent experiments. (E) pmaxGFP transfection efficiency by nucleofection on stored neutrophils (pre-conditioning and storage for 20 h post-transfection) or in the absence of treatment (+O₂ conditions). (E-F) Flow cytometry quantification of the GFP+ neutrophil proportion within the whole neutrophil population (E) or within viable (Annexin V-/PI-) population (F). Results are averaged from three independent experiments (n=3) performed in triplicate. Error bars indicate SD. *** indicates P < 0.001.

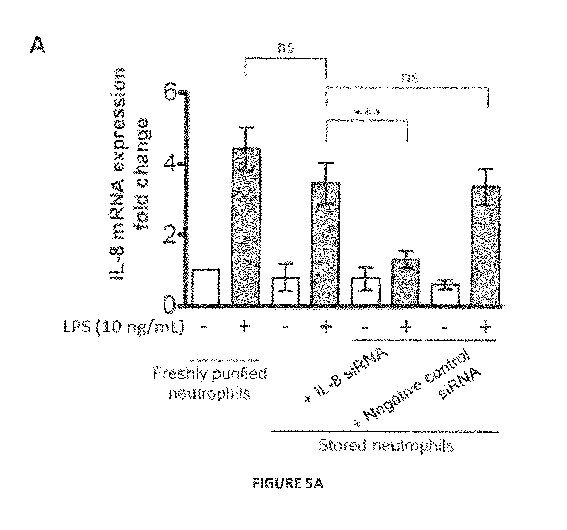

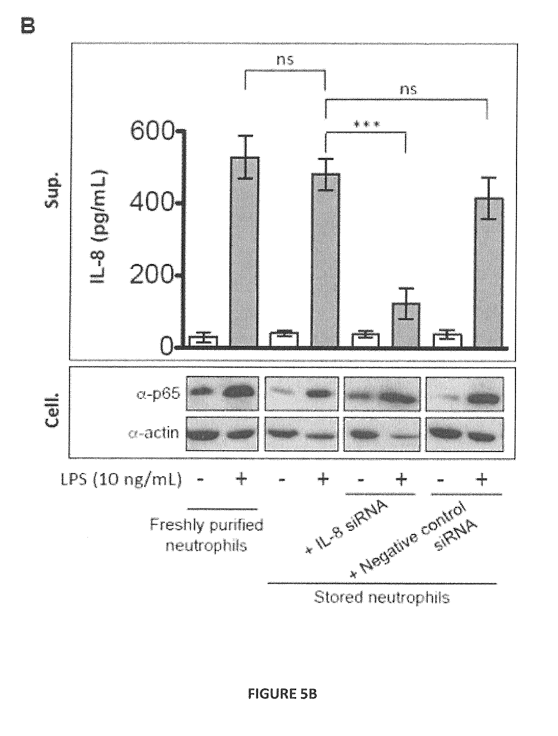

**Figure 5****. Neutrophil storage allows IL-8 siRNA gene silencing by nucleofection.** (A) qRT-PCR analysis of IL-8 mRNA expression in freshly purified neutrophils (5.10⁵ cells) upon storage (-O₂, + 3mM glucose + 32 µg/mL DMOG). Stored neutrophils were nucleofected with IL-8 siRNA or Negative control siRNA when indicated. Neutrophils were either untreated (whit bars) or stimulated with 10 ng/mL LPS (grey bar) during 3 h at 37°C. IL-8 mRNA expression fold change were calculated as compared to untreated freshly purified neutrophil. Error bars indicate SD. 'ns' indicates P > 0.05, *** indicates P < 0.001, (n=3). (B) IL-8 release in the supernatant fractions (Sup.) by neutrophils stored as described in (A) and stimulated with 10 ng/mL LPS for 3 h at 37°C. IL-8 quantifications were performed by ELISA (5.10⁵ cells), Error bars indicate SD. 'ns' indicates P > 0.05, *** indicates P < 0.001, (n=3). As controls, p65 and actin were detected by Western blot in cellular (Cell.) fractions with specific antibodies. Results are representative of three independent biological samples.
**Figure 6****. Viable neutrophils purification from apoptotic neutrophils and red blood cells (RBCs) by flow cytometry.** (A) Neutrophils purified with Method 2 (citrated venous blood) were contaminated by RBC at final purification step. Samples were additionally purified by negative selection using CD235a (glycophorin) microbeads, allowing RBC retention. (B) Upon storage, apoptotic neutrophils were eliminated using Annexin V microbeads, allowing negative selection of viable cells. Experiments are representative of at least five individual experiments.

### EXAMPLES

The examples have been performed using the following materials and methods:
Ethical approval
   All participants gave written informed consent in accordance with the Declaration of Helsinki principles. Peripheral Human blood was collected from healthy patients at the ICAReB service of the Pasteur Institute (authorization DC No.2008-68).
Human blood collection
   Human blood was collected from the antecubital vein into tubes containing sodium heparinate (1% final concentration) (Method 1) or sodium citrate (3,8% final) (Method 2) as anticoagulant molecules (see below).
Neutrophils isolation
   Immediately after blood collection, neutrophils were isolated from total blood samples following two different methods as described below.
   *Method 1*. Heparinized venous blood was layered over 7 ml Histopaque^{®}1119 (Sigma-Aldrich) and centrifuged at 800 *x g* for 20 min. at room temperature (RT). Plasma and the mononuclear cell layer were discarded. The diffuse red phase containing neutrophils, above Red Blood Cell (RBC) pellet, was collected and washed with RPMI 1640 (without glutamine and Phenol Red) (Gibco Invitrogen) with 10 mM Hepes and layered on 10 ml of discontinuous Percoll^{®} Gradient (GE Healthcare) prepared in Ca²⁺/Mg²⁺-free phosphate buffered saline (PBS) with final Percoll^{®} concentrations of 65, 70, 75, 80 and 85%. After centrifugation at 800 *x g* for 20 min, neutrophil buffy coat located at the 70/75% interface was collected and washed in RPMI 1640 with 10 mM Hepes before culture. Neutrophils were resuspended at 1x10⁶ cells/ml in RPMI 1640 with L Glutamine and 25 Mm Hepes (Gibco, Life Technologies), 10% of heat inactivated Foetal Bovine Serum (FBS) (Invitrogen, Gibco).
   *Method 2.* Citrated venous blood (45 mL) was centrifuged at 445 g for 15 min. at room temperature. Platelet rich plasma (PRP) was collected and centrifuged at 2500 x g for 20 min to form platelet poor plasma (PPP). 6 ml of 6% dextran were added to cells, to up to 50 ml with sterile saline (0.9% NaCl). Cells were subsequently mixed gently by inverting tubes. After red blood cells sedimentation, the upper layer containing neutrophils was transferred to clean 50 ml falcon tubes and centrifuged at 239 *x g* for 6 min. Pellet was resuspended in 1 ml of platelet poor plasma (PPP) and deposited on a Percoll^{®} gradient with a lower phase (51% Percoll^{®}+ 49% PPP) and an upper phase (42% Percoll^{®}+ 58% PPP). After centrifugation at 239 *x g* for 15 min, the granulocyte layer was washed in 25% PPP (10 ml PPP + 30 ml Hanks without Ca²⁺ and Mg²⁺) and cells were counted.
   In order to increase the purity of the neutrophils enriched fraction, remaining red blood cells and dead cells were removed as followed. Neutrophils fraction was pelleted by centrifugation (300 *x g,* 20°C, 10 min) and re-suspended in 100 µl of Dead Cell Removal MicroBead (Miltenyi Biotec, Auburn, CA) and incubated at room temperature in the dark for 15 min. Isolation of viable neutrophils was performed in LS columns and a midi MACS® separator (Miltenyi Biotech) following the protocol provided by the manufacturer. Briefly, enriched cells from the first step were re-suspended in 80 µl of 1X Binding buffer and labelled with 20 µl of Annexin V MicroBead (Miltenyi Biotec). After 15 min incubation at 8°C, cells were transferred in new LS columns. Viable unlabelled neutrophils (Annexin V-) were subsequently collected. Red blood cells were subsequently removed by negative selection using CD235a (glycophorin) microbeads (Miltenyi Biotec), following the manufacturer's recommendations.
Culture media
   Human purified neutrophils were cultured in RPMI 1640 (Life Technologies) supplemented with 10 mM Hepes or in RPMI 1640 with L Glutamine, 25 mM Hepes (Gibco, Life Technologies) and 10% of heat inactivated Foetal Bovine Serum (FBS) (Invitrogen, Gibco) when indicated.
   *Shigella flexneri* 5a (*S. flexneri*) was grown in trypticase soy (TCS) broth or on TCS agar plates supplemented with 0.01% Congo Red (Sigma).
Neutrophils storage conditions
   Neutrophils fractions with a >95% purity obtained by applying Method 1 or Method 2 were resuspended at a 5x10⁶ cells/mL concentration in RPMI 1640 with L Glutamine and 25 mM Hepes (Gibco, Life Technologies), 10% of heat inactivated Foetal Bovine Serum (FBS) (Invitrogen, Gibco) and indicated concentrations of glucose (Sigma-Aldrich) and Dimethyloxalylglycine or DMOG (Cayman Chemical Company).
   For comparative experiments aiming at characterizing the role of oxygen on neutrophils survival, purified neutrophils (Method 1 or Method 2) were split in two identical fractions. One was stored at 37°C with 5% CO₂ under atmospheric conditions (20% O₂ condition, +O₂). The second one was stored at 37°C in an anoxic chamber (Minimacs, Don Withley) under an atmosphere containing 90% N₂, 5% CO₂ and 5% H₂ (0% O₂ condition, -O₂), during indicated time.
Antibodies
   For immunofluorescence, MMP-9 was detected using a rabbit polyclonal antibody (Novus Biologicals, NBP1-45719).
   For flow cytometry, apoptotic cells were labelled with (allophycocyanin) APC-Annexin V (BD Bioscience). Cell surface exposed antigens were labelled with (phycoerythrin) PE Mouse anti-human CD16 (clone 3G8, BD Pharmingen), APC mouse anti-human CD11b (clone D12, BD Biosciences), PE mouse anti-human CD66b (clone G10F5, BD Pharmingen), PE, mouse anti-human CD15 (clone W6D3, BD Pharmingen) or FITC mouse anti-human CD63 (clone H5C6, BD Pharmingen). For western blot analysis, a monoclonal α-IL-8 antibody (SAB1409243, Sigma-Aldrich), a rabbit polyclonal α-p65 antibody (ab7970, Abcam) and a rabbit polyclonal α-actin antibody (A2066, Sigma-Aldrich) were used.
Flow cytometry
   *Cell viability.* For neutrophil survival analysis, human neutrophils (1x10⁶) were washed twice in PBS and then resuspended in 1ml of Annexin V binding Buffer. A 100 µl sample (1x10⁵ cells) was stained with 5 µl of APC-Annexin V and 5 µl of Propidium iodide (PI) (0.5 µg/ml final concentration) as recommended by the manufacturer. At least 10⁴ events were acquired for each condition on a FACSCalibur™ flow cytometer (BD Biosciences), and data were analyzed using CellQuest™ Pro Software (BD Biosciences). Viable cells were defined as Annexin V-/PI- quantifications.
   *Neutrophil degranulation assay.* For neutrophil degranulation assays, 2.10⁶ neutrophils purified with Method 2 were stimulated with *S. flexneri* at MOI 20. After 10 min centrifugation at 300 x g, infected neutrophils were incubated during 15 min at 37°C. Cell surface markers exposure was quantified by flow cytometry as follows: 2.5x10⁵ cells were collected and incubated with 5 µL of PE Mouse anti-human CD16, APC mouse anti-human CD11b, PE mouse anti-human CD66b, PE, mouse anti-human CD15 or FITC mouse anti-human CD63. Cell labelling was analysed using a FACSCalibur™ flow cytometer (BD Biosciences), and data were analysed using CellQuest™ Pro Software (BD Biosciences). Results are representative of two independent experiments performed.
*Plasmid or siRNA nucleofection*
   Plasmid or siRNA transfection were performed by nucleofection (Lonza). Neutrophils purified with Method 2 (2.10⁶ cells/transfection) were pre-incubated 2 hours in 500 µL of supplemented culture media (RPMI + 10% SVF + 3 mM glucose + 32 µg/mL DMOG) at 37°C under anoxic conditions. Cells were collected by centrifugation (300 *x g,* 10 min at room temperature). Cells were washed in RPMI only. After an additional centrifugation, pellets were carefully resuspended in 100 µl supplemented Mouse T cell Nucleofector® solution (Lonza). 2 µg of pmaxGFP plasmid (Lonza) or 2 pmole of siRNA (SMARTpool®: Accell IL8 siRNA (Thermo Fischer Scientific) or Negative control siRNA (Qiagen)) were added before transferring the mixture into certified nucleofection cuvettes. DNA transfer was performed by electroporation using Nucleofector Program Y-01 (Amaxa® Nucleofector® device (Lonza)). Transfected samples were immediately transferred first in 500 µL of pre-equilibrated supplemented culture media and subsequently into 24-well plate containing 1 mL of pre-equilibrated supplemented culture media. 20 h post-transfection, transfected cells were analyzed by flow cytometry, fluorescence imaging (plasmid); quantitative PCR, ELISA or Western blot (siRNA).
*Statistical analysis*
   Data were analysed using Prism 5.0 software (GraphPad) using Student's T-test. Significance was accepted when P<0.05.
*Western Blot*
   Neutrophils stimulated with LPS or *S. flexneri* (see above) cellular extract and supernatants were incubated with anti-protease Cocktail (Roche). Proteins were separated by electrophoresis on SDS-Page gel and transferred onto nitrocellulose membrane (Life technologies) and blocked in PBS milk 5% for 1 hour at room temperature. Membranes were further incubated with α-p65 and α-actin primary antibodies (1:1000) overnight: antibody binding was detected with chemiluminescence (ECL kit, GE Healthcare) using conjugated antibodies to (Horseradish peroxidase) HRP (Dako) (1:5000).
*Immunofluorescence and Imaging*
   Immunofluorescence detection was performed in PBS/Saponin 0.1% using a rabbit α-MMP-9 (1:1000) and α-rabbit-FITC conjugated secondary antibodies (1:1000). Actin was stained with phalloidin-Rhodamin (1:1000), DNA was stained with Dapi (1:1000).
   Fluorescent-labelled cells were observed using a laser-scanning TCS SP5 confocal microscope (Leica). Image analysis was performed using Fiji software.
*Phagocytosis assay*
   *Shigella flexneri* pGFP (M90T pGFP) or *S*. *aureus* pGFP strains were grown until an OD₆₀₀=0.5 was reached and were incubated with purified human neutrophils in RMPI 1640 with 10 mM Hepes at a MOI 20 with inactivated human serum and centrifuged at 300 x g during 10 min. An additional incubation of 15 min at 37°C was performed to allow the phagocytosis of bacteria. For immunofluorescence staining, after three washes in PBS, infected cells were fixed with paraformaldehyde (PFA) 3% during 30 min. After an additional washing in PBS, infected cells were stained as described below. For intracellular bacteria counting, infected cells were washed three times with PBS and incubated with 50 µg/mL gentamycin for 30 min at 37°C and further washed three times in PBS. Intracellular bacteria were counted after cells were lysed in 1% saponin.
*NET formation assay*
   Purified Neutrophils (5.10⁵ per well) were seeded on glass coverslips treated with 0.001% polylysine, centrifuged at 300 x g for 10 min and treated 25 nM PMA (phorbol 12-myristate 13-acetate) (Sigma-Aldrich) for 3 hours at 37°C. Cells were subsequently fixed with 4% PFA prior immunofluorescence staining.
*RNA extraction*
   IL-8 expression induction was performed on 2.10⁵ human neutrophils either freshly purified, stored 20h in anoxic conditions with 3 mM glucose and 32 µg/mL DMOG, or stored and transfected 20 h with IL-8 siRNA or Negative control siRNA (see above). Neutrophils IL-8 expression was induced with 10 ng/mL *E. coli* LPS (InvivoGen-Cayla) for 180 min. RNAs were extracted with RNeasy Mini kit (Qiagen) from each condition; total RNAs were extracted from three independent biological samples.
*Transcriptional analysis*
   Following RNAs extraction, cDNAs were produced with *SuperScript II Reverse Transcriptase* (Invitrogen) and oligo(dT)12-18 primer (Invitrogen) as recommended by the supplier. The following primers (purchased from Invitrogen) were used: *IL-8,* 5'-GCCTTCCTGATTTCTGCAGC-3' (SEQ ID No: 1) and 5'-TGCACCCAGTTTTCCTTGG-3' (SEQ ID No: 2); *gapdh* 5'-TCGCTCTCTGCTCCTCC-3' (SEQ ID No: 3) and 5'-TTAAAAGCAGCCCTGGTGAC-3' (SEQ ID No: 4). qPCR reactions were run on an ABI 7900HT (Applied Biosystems) using Power SYBR® Green mix (Applied Biosystems) according to the manufacturer's instructions. *gapdh* was used as an internal control gene to obtain relative expression compared to untreated freshly purified neutrophils and expressed as IL-8 mRNA expression fold change. Data were analysed with SDS 2.2 software (Applied Biosystems). Means and SD were calculated from three independent samples and performed in triplicate.
*ELISA*
   Release of IL-8 was determined by ELISA as described previously (Hattar et al, 2001). Mouse monoclonal α-IL-8 antibody (4 µg/mL) was bound to 96-well ELISA plates at 4°C overnight and washed 5 times with PBS, 0.05% Tween. Wells were blocked in PBS, 0.05% Tween, 0.1% BSA for 1h at room temperature and washed 5 times with PBS, 0.05% Tween. Neutrophil supernatants (concentrated to reach 100 µg/mL) were bound to α-IL-8 coated plates (100 µL, 10 µg total protein) at room temperature during 2h and washed 5 times with PBS, 0.05% Tween. Recombinant human IL-8 (Sigma-Aldrich) was used for standard titration curves. IL-8 was detected with mouse monoclonal α-IL-8 antibody followed by phosphatase alkaline goat α-mouse conjugated antibody (Beckman Coulter) (200 ng per well). Antibodies diluted in PBS, 0.05% Tween were incubated with the plates for 90 min. Following washes, substrate PNPP (0.1%) was added in a Tris HCl 0.1M pH 9 with NaCl 4.5M buffer to each well and incubated for 60 min at room temperature. Absorbance was measured at 405 nm on a Sunrise microplate reader (Tecan). Mean and SD were calculated from experiments performed in triplicate on three independent biological samples. IL-8 concentration (pg/mL) was calculated from standard curve.

### Example 1: Neutrophils survival increases upon synergistic action of glucose and DMOG supplementation in anoxic conditions

In this study, neutrophil survival rate was evaluated in various conditions by flow cytometry, by quantifying the proportion of viable (propidium iodide negative staining, PI-) and non-apoptotic (Annexin V negative staining, Annexin V-) cells.

In routine, heparin and citrate are the most commonly used anticoagulants for clinical analysis or *in vitro* basic research^{6,22}. Heparin acts as an anticoagulant by activating antithrombin²³. The inventors demonstrated that neutrophils purified with heparin (Method 1) were viable upon collection and purification in the presence of atmospheric oxygen (20% O₂, +O₂) (89±4%, Figure 1A). However, a drastic decrease of their survival rate was observed as soon as 20 h (16±7% *vs*. 89±4%, P < 0.001) (Figure 1A). Conversely, under anoxic conditions (0% O₂, -O₂), neutrophils survival rate 20 h post purification was significantly higher than in the presence of oxygen (35±11% *vs.* 16±7%, P < 0.01) (Figure 1A,B) but remained limited. Glucose supplementation (3mM) did not have any effect on survival rate in the presence of oxygen at 20 h or 48 h (P > 0.05) (Figure 1A), but a significant increase was observed after 20 h storage in anoxia (P < 0.05) (Figure 1B). This effect was no longer observed after 48 h storage (P > 0.05) (Figure 1B). Low oxygen level and high glucose concentration synergistic effect on neutrophils survival may be the consequence of anaerobic glycolysis optimization, since glycolysis is the main energy production source of neutrophils^{20,21}.

Dimethyloxalylglycine (DMOG) is a pan-hydroxylase inhibitor stabilizing HIF-1α²⁴, which was shown to decrease neutrophil apoptosis²⁵. Its effect on neutrophil survival in the absence of oxygen with 3mM glucose supplementation was evaluated in a concentration-dependent manner. Various DMOG concentrations (8 to 40 µg/mL) were assessed; a significant survival rate increase was observed after 20 h and 48 h storage with 32 µg/mL DMOG (P < 0.05, Fig. 1C), reaching 54±10% and 14±10% survival rates respectively.

### Example 2: Neutrophils isolation with citrate increases cell survival rates as compared to heparin

Anticoagulant molecule used for blood collection impacts on neutrophil survival and may have diverse effects on neutrophils activation⁶.

Citrate impairs several enzymes involved in the coagulation cascade, through calcium-chelating property²². As reported previously, although heparin is widely used, it has been shown to activate neutrophils as a side effect²⁶, whereas citrate limits neutrophils activation⁶ and was assessed in similar storage conditions.

Neutrophils purified with citrate (Method 2) were subsequently separated from remaining red blood cells with CD235a Microbeads (Figure 6). As a first statement, collecting blood in the presence of citrate allowed a significant increase of neutrophils survival rate as compared to heparin in the presence or in the absence of oxygen 20 h- and 48 h-post purification (P < 0.01) (Fig. 2A,B). As described with heparin, supplementation with 3 mM glucose increased neutrophils survival 20 h- and 48 h-post purification when stored under anoxic conditions (P < 0.01) (Figure 2A), which was not observed when stored in the presence of oxygen (p>0.05) (Figure 2A). Synergistic effect of anoxia and 3 mM glucose supplementation resulted in survival rates of 64±8% and 41±3%, 20 h- and 48 h-post purification (Figure 2A), which further increased to 70±8% (P < 0.05) and 49±2% (P < 0.05) upon addition of 32 µg/mL DMOG, respectively (Figure 2B). These results demonstrate neutrophils survival rate optimization was due to a synergistic effect of anoxia, supplementation with 3 mM glucose and 32 µg/mL DMOG (hereinafter referred to as 'storage conditions' or 'storage').

### Example 3: Stored neutrophils remain functional

The above results demonstrated that neutrophils purified on citrate and stored for 20 h or 48 h under anoxic conditions with 3 mM glucose and 32 µg/mL DMOG remain mainly viable. To determine if viable neutrophil population remain functional, its ability to phagocytose bacteria was analysed, as well as its ability to form NET upon PMA stimulation and to release granule components as compared to freshly purified neutrophils.

After 20 h or 48 h storage, viable neutrophils were purified by negative selection on Annexin V binding Microbeads to obtain a pure Annexin V-/PI- neutrophil population (Figure S1B). As shown in Figure 3A, purified viable neutrophils obtained after 20 h or 48 h storage phagocytosed *S. flexneri* with the same efficiency as freshly purified neutrophils (P > 0.05). In addition, they remained sensitive to PMA-dependent NET formation (Figure 3B). To evaluate neutrophil degranulation efficiency of stored viable neutrophils as compared to freshly purified neutrophils, the inventors quantified by flow cytometry cell-surface exposure of granule markers upon contact with *S*. *flexneri;* CD63 is a primary (azurophil) granule marker²⁷, CD66b is specifically localised in secondary (specific) granules²⁸ and CD11b is a tertiary (gelatinase) granule marker²⁹. CD16 (or FcgR IIIb) is present in secretory vesicles, but also on naïve neutrophils surface³⁰.

The inventors first showed that granule markers detection on neutrophil surface (CD63, CD66b, CD11b and CD16) was comparable on naïve neutrophils either freshly purified or upon 20 h storage (Figure 3C). These results demonstrated that if CD63 and CD11b were not detected, conversely CD66b and CD 16 were significantly exposed on neutrophils surface upon collection and purification. They then demonstrated that, in the presence of *S*. *flexneri,* all types of granules markers were similarly detected either on freshly purified neutrophils or upon 20 h storage (Figure 3C). *S. flexneri* stimulation increased CD63, CD66b and CD11b signal detection (Figure 3C). CD16 abundance decreased upon stimulation, both on freshly purified neutrophils and upon storage (Figure 3C), probably due to its recruitment and internalisation upon bacteria phagocytosis. These results altogether demonstrate that viable stored neutrophils remain functional and their bactericidal properties are not altered, as compared to freshly purified neutrophils.

### Example 4: Neutrophils storage allows optimized DNA transfection by nucleofection

To date, DNA transfection into neutrophils remained difficult due to the short lifespan of purified cells under atmospheric conditions. It has recently been demonstrated that nucleofection was a successful approach to transfect plasmids into neutrophils^{31,32}, although the efficiency remained limited to 5% of total cells; nucleofection *per se* lead to 8% of PI+ neutrophils. The inventors hypothesized that storing neutrophils prior to transfection would result in enhancement of nucleofection efficiency and cell survival.

Prior to nucleofection, neutrophils were pre-conditioned in storage conditions; transfected cells remained stored for 20 h in similar conditions immediately after nucleofection. The inventors demonstrated that pmaxGFP plasmid could be efficiently transfected in neutrophils by nucleofection, using Y-01 program (Fig. 4). Upon nucleofection, 20 h post-transfection, the majority of neutrophils remained viable (51% Annexin V-/PI-, representative experiment; Figure 4A) as observed upon storage only (76% Annexin V-/PI-, representative experiment; Figure 4A). Only 10% of nucleofected neutrophils were PI+, consistently with previous reports (8%)^{31,32}. Without storage conditions (+O₂), Annexin V-/PI- neutrophils proportion decreased drastically (24% *vs*. 51 %, representative experiment; Figure 4A). This result demonstrates that conditioning neutrophils is required for nucleofection optimization. The inventors could detect pmaxGFP expression in 36.7% of the whole neutrophil population, including 38.6% of the Annexin V-/PI- neutrophil population (representative experiment, Figure 4B,C,D). Nucleofection efficiency was stable through independent replicates and averaged at 38±7% of the whole neutrophil population and 37±6% of the Annexin V-/PI- neutrophil population (n=3, Figure 4E,F). Without storage, neutrophil nucleofection efficiency remained low in both the whole and the Annexin V-/PI- neutrophil populations (11±2% and 7±2%), consistently with previous reports^{31,32}. In conclusion, pre-conditioning and storing neutrophils allowed a significant increase of nucleofection efficiency (P < 0.001; Figure 4E,F).

### Example 5: Neutrophils storage allows siRNA gene silencing by nucleofection

Beyond optimization of plasmid transfection efficiency, the inventors aimed at determining if storing neutrophils would allow siRNA gene silencing by nucleofection. To do so, they assessed the IL-8-siRNA neutrophil nucleofection effect on the well-characterized induction of IL-8 expression and release upon lipopolysaccharide (LPS) stimulation^{33,34}. Neutrophils responsiveness was assessed on freshly purified cells, stored neutrophils (20 h) and transfected neutrophils stored for 20 h post-transfection (IL-8 siRNA or Negative control siRNA). By qRT-PCR, they observed that neutrophils storage did not modulate significantly IL-8 expression upon LPS (10 ng/mL) stimulation during 3 h, as compared to stimulated freshly purified cells (P > 0.05, Fig. 5A). Transfection of IL-8 siRNA was efficient 20 h post-nucleofection, as compared to stimulated stored neutrophils control; a significant reduction of IL-8 mRNA expression was observed (P < 0.001, Fig. 5A), which was not observed with negative control siRNA (P > 0.05, Fig. 5A).

To further confirm the efficiency of IL-8 gene silencing, IL-8 release by neutrophils stimulated with LPS for 3 h was quantified. As control, in all conditions tested, an increase of p65 expression was observed upon LPS stimulation (Fig. 5B) as previously reported^{35,36}. Consistently with gene expression regulations (Fig. 5A), the inventors observed that LPS-dependent IL-8 release was significantly reduced upon IL-8 siRNA transfection as compared to untreated stored neutrophils (P < 0.001, Fig. 5B). No change was observed upon neutrophils storage as compared to freshly purified cells or upon negative control siRNA transfection as compared to stored cells (P > 0.05, Fig. 5B). These results demonstrate that storing neutrophils allows efficient and functional siRNA transfection in neutrophils.

### Discussion

Neutrophils have long been known as "short-lived cells"³⁷. This assumption is mostly based on inappropriate storage conditions *in vitro* rather than on their lifespan in humans, which appears to be longer as previously expected¹⁷. Neutrophil global lifespan in humans should also include their period of storage in the bone marrow, which lasts 4-6 additional days^{15,13}. Previous reports describing improvement in conservation of viable and functional neutrophils *in vitro* did not lead to technical improvement of neutrophil genetic manipulation (plasmid or siRNA transfection)^{38,39}. Until now, plasmid transfection was described by nucleofection but appeared not to be efficient, with less than 5% transfected cells^{31,32}.

In the experiments described above, the inventors defined optimized storage conditions based on neutrophil adaptation to low oxygen conditions, as observed during their maturation in the bone marrow⁷ but also recently highlighted *in vitro* under the control of HIF^{19,25}. They demonstrated that in conditions of (i) anoxia, (ii) glycolysis promotion by glucose supplementation (3 mM) and (iii) HIF stabilization in the presence of DMOG (32 µg/mL), neutrophil survival could be extended up to 70±8% after 20 h and 49±2% after 48 h storage (Fig. 1C and 2B). Moreover, neutrophils bactericidal activities were not affected by storing cells for 20 h in these conditions as compared to freshly purified cells; since their ability to phagocytose bacteria, to produce NETs upon PMA stimulation or to release granules upon *S. flexneri* infection remained intact (Fig. 4).

As stored neutrophils were functional, genetic manipulation could be envisaged, such as plasmid and siRNA transfection. The inventors demonstrated that pre-conditioning neutrophils increased the efficiency of plasmid transfection by nucleofection, as 37% of viable neutrophils were transfected (Fig. 4F). This will allow further studies focusing on neutrophil fundamental physiology but also on neutrophil defence mechanisms subversion by bacteria virulence effector as described in other infected cell types⁴⁰.

As so far, gene silencing was not permitted on purified neutrophils exposed to atmospheric conditions, its efficiency was assessed on neutrophils pre-conditioned and stored in preserving conditions herein described. Targeting IL-8 mRNA through IL-8 siRNA transfection reduced significantly LPS-induced level of expression (Fig. 5A). IL-8 siRNA transfection decreased IL-8 release by stored neutrophils upon LPS stimulation (Fig. 5B). Broad range of potential applications of siRNA transfection allowed by storage will promote further investigations on neutrophils physiology and antimicrobial activities, including siRNA high-throughput screening and shRNA introduction through transient transfection.

### REFERENCES

1. Serhan CN, Savill J. Resolution of inflammation: the beginning programs the end. Nat. Immunol. 2005;6(12):1191-1197.
2. Savill J. Apoptosis in resolution of inflammation. J. Leukoc. Biol. 1997;61(4):375-380.
3. Maianski NA, Roos D, Kuijpers TW. Bid truncation, bid/bax targeting to the mitochondria, and caspase activation associated with neutrophil apoptosis are inhibited by granulocyte colony-stimulating factor. J. Immunol. 2004; 172(11):7024-7030.
4. Kobayashi SD, Voyich JM, Whitney AR, DeLeo FR. Spontaneous neutrophil apoptosis and regulation of cell survival by granulocyte macrophage-colony stimulating factor. J. Leukoc. Biol. 2005;78(6):1408-1418.
5. Strauss RG. Role of granulocyte/neutrophil transfusions for haematology/oncology patients in the modern era. Br. J Haematol. 2012; 158(3):299-306.
6. Freitas M, Porto G, Lima JLFC, Fernandes E. Isolation and activation of human neutrophils in vitro. The importance of the anticoagulant used during blood collection. Clin. Biochem. 2008;41(7-8):570-575.
7. Sigurdsson F, Khanna-Gupta A, Lawson N, Berliner N. Control of late neutrophil-specific gene expression: insights into regulation of myeloid differentiation. Semin. Hematol. 1997;34(4):303-310.
8. Eliasson P, Jönsson J-I. The hematopoietic stem cell niche: low in oxygen but a nice place to be. J. Cell. Physiol. 2010;222(1):17-22.
9. Simsek T, Kocabas F, Zheng J, et al. The distinct metabolic profile of hematopoietic stem cells reflects their location in a hypoxic niche. Cell Stem Cell. 2010;7(3):380-390.
10. Epstein AC, Gleadle JM, McNeill LA, et al. C. elegans EGL-9 and mammalian homologs define a family of dioxygenases that regulate HIF by prolyl hydroxylation. Cell. 2001;107(1):43-54.
11. Bruick RK, McKnight SL. A conserved family of prolyl-4-hydroxylases that modify HIF. Science. 2001;294(5545):1337-1340.
12. Maxwell PH, Wiesener MS, Chang GW, et al. The tumour suppressor protein VHL targets hypoxia-inducible factors for oxygen-dependent proteolysis. Nature. 1999;399(6733):271-275.
13. Dancey JT, Deubelbeiss KA, Harker LA, Finch CA. Neutrophil kinetics in man. J. Clin. Invest. 1976;58(3):705-715.
14. Pittman RN. Oxygen gradients in the microcirculation. Acta Physiol (Oxf). 2011;202(3):311-322.
15. ATHENS JW, HAAB OP, RAAB SO, et al. Leukokinetic studies. IV. The total blood, circulating and marginal granulocyte pools and the granulocyte turnover rate in normal subjects. J. Clin. Invest. 1961;40:989-995.
16. Peters AM, Roddie ME, Danpure HJ, et al. 99Tcm-HMPAO labelled leucocytes: comparison with 111In-tropolonate labelled granulocytes. Nucl Med Commun. 1988;9(6):449-463.
17. Pillay J, Braber den I, Vrisekoop N, et al. In vivo labeling with 2H2O reveals a human neutrophil lifespan of 5.4 days. Blood. 2010;116(4):625-627.
18. Rankin SM. The bone marrow: a site of neutrophil clearance. J. Leukoc. Biol. 2010;88(2):241-251.
19. Walmsley SR, Chilvers ER, Thompson AA, et al. Prolyl hydroxylase 3 (PHD3) is essential for hypoxic regulation of neutrophilic inflammation in humans and mice. J. Clin. Invest. 2011;1211(3):1053-1063.
20. Borregaard N, Herlin T. Energy metabolism of human neutrophils during phagocytosis. J. Clip. Invest. 1982;70(3):550-557.
21. Maianski NA, Geissler J, Srinivasula SM, et al. Functional characterization of mitochondria in neutrophils: a role restricted to apoptosis. Cell Death Differ. 2004;11(2):143-153.
22. Lee G, Arepally GM. Anticoagulation techniques in apheresis: from heparin to citrate and beyond. J Clin Apher. 2012;27(3):117-125.
23. Engstad CS, Gutteberg TJ, Osterud B. Modulation of blood cell activation by four commonly used anticoagulants. Thromb. Haemost. 1997;77(4):690-696.
24. Jaakkola P, Mole DR, Tian YM, et al. Targeting of HIF-alpha to the von Hippel-Lindau ubiquitylation complex by 02-regulated prolyl hydroxylation. Science. 2001;292(5516):468-472.
25. Walmsley SR, Print C, Farahi N, et al. Hypoxia-induced neutrophil survival is mediated by HIF-1alpha-dependent NF-kappaB activity. J. Exp. Med. 2005;201(1):105-115.
26. Brown RA, Leung E, Kankaanranta H, Moilanen E, Page CP. Effects of heparin and related drugs on neutrophil function. Pulm Pharmacol Ther. 2012;25(2):185-192.
27. Pols MS, Klumperman J. Trafficking and function of the tetraspanin CD63. Exp. Cell Res. 2009;315(9):1584-1592.
28. Jog NR, Rane MJ, Lominadze G, et al. The actin cytoskeleton regulates exocytosis of all neutrophil granule subsets. Am. J. Physiol., Cell Physiol. 2007;292(5):C 1690-700.
29. Mollinedo F, Nakajima M, Llorens A, et al. Major co-localization of the extracellular-matrix degradative enzymes heparanase and gelatinase in tertiary granules of human neutrophils. Biochem. J. 1997;327 (Pt 3):917-923.
30. Faurschou M, Borregaard N. Neutrophil granules and secretory vesicles in inflammation. Microbes Infect. 2003;5(14):1317-1327.
31. Johnson JL, Ellis BA, Munafo DB, Brzezinska AA, Catz SD. Gene transfer and expression in human neutrophils. The phox homology domain of p47phox translocates to the plasma membrane but not to the membrane of mature phagosomes. BMC Immunol. 2006;7:28.
32. Magalhães MAO, Zhu F, Sarantis H, et al. Expression and translocation of fluorescent-tagged p21-activated kinase-binding domain and PH domain of protein kinase B during murine neutrophil chemotaxis. J. Leukoc. Biol. 2007;82(3):559-566.
33. Strieter RM, Kunkel SL, Showell HJ, et al. Endothelial cell gene expression of a neutrophil chemotactic factor by TNF-alpha, LPS, and IL-1 beta. Science. 1989;243(4897):1467-1469.
34. Hattar K, Fink L, Fietzner K, et al. Cell density regulates neutrophil IL-8 synthesis: role of IL-1 receptor antagonist and soluble TNF receptors. J. Immunol. 2001;166(10):6287-6293.
35. McDonald PP, Bald A, Cassatella MA. Activation of the NF-kappaB pathway by inflammatory stimuli in human neutrophils. Blood. 1997;89(9):3421-3433.
36. Miskolci V, Rollins J, Vu HY, et al. NFkappaB is persistently activated in continuously stimulated human neutrophils. Mol. Med. 2007;13(3-4):134-142.
37. Cassatella MA, Locati M, Mantovani A. Never underestimate the power of a neutrophil. Immunity. 2009;31(5):698-700.
38. Hubel K, Rodger E, Gaviria JM, et al. Effective storage of granulocytes collected by centrifugation leukapheresis from donors stimulated with granulocyte-colony-stimulating factor. Transfusion. 2005;45(12):1876-1889.
39. Price TH, Dale DC. Neutrophil transfusion: effect of storage and of collection method of neutrophil blood kinetics. Blood. 1978;51(5):789-798.
40. Phalipon A, Sansonetti PJ. Shigella's ways of manipulating the host intestinal innate and adaptive immune system: a tool box for survival? Immunol Cell Biol. 2007;85(2):119-129.
41. Witko-Sarsat V, Mocek J, Bouayad D, et al. Proliferating cell nuclear antigen acts as a cytoplasmic platform controlling human neutrophil survival. J. Exp. Med. 2010;207(12):2631-2645.
42. Rossi AG, Sawatzky DA, Walker A, et al. Cyclin-dependent kinase inhibitors enhance the resolution of inflammation by promoting inflammatory cell apoptosis. Nat. Med. 2006;12(9):1056-1064.
43. Li Y, Prasad A, Jia Y, et al. Pretreatment with phosphatase and tensin homolog deleted on chromosome 10 (PTEN) inhibitor SF1670 augments the efficacy of granulocyte transfusion in a clinically relevant mouse model. Blood. 2011;117(24):6702-6713.
44. Zhu D, Hattori H, Jo H, et al. Deactivation of phosphatidylinositol 3,4,5-trisphosphate/Akt signaling mediates neutrophil spontaneous death. Proc. Natl. Acad. Sci. U.S.A. 2006;103(40):14836-14841.
45. Gabelloni ML, Trevani AS, Sabatté J, Geffner J. Mechanisms regulating neutrophil survival and cell death. Semin Immunopathol. 2013;35(4):423-437.
46. Katja Hattar, Ludger Fink, Karin Fietzner, Barbara Himmel, Friedrich Grimminger, Werner Seeger and Ulf Sibelius, Cell Density Regulates Neutrophil IL-8 Synthesis: Role of IL-1 Receptor Antagonist and Soluble TNF Receptors, The Journal of Immunology, May 15, 2001, vol. 166 no. 10 6287-6293

## Claims

1. A method for keeping leukocytes alive *ex vivo* or *in vitro,* comprising maintaining the leukocytes in a medium comprising from 3 to 10 mM of glucose, in hypoxic conditions with P(O₂) ≤ 10 mM Hg.

2. The method of claim 1, wherein P(O₂) ≤ 1 mM Hg.

3. The method of claim 1 or claim 2, wherein the glucose concentration is comprised between 3 and 6 mM.

4. The method of any of claims 1 to 3, wherein said medium is plasma.

5. The method of any of claims 1 to 3, wherein said medium is a culture medium comprising an effective amount of a compound that stabilizes the hypoxia inducible factor-1 (HIF-1).

6. The method of claim 5, wherein said compound that stabilizes the hypoxia inducible factor-1 (HIF-1) comprises or consists of a prolyl hydroxylase inhibitor.

7. The method of claim 6, wherein the compound that stabilizes HIF-1α is the dimethyloxalylglycine (DMOG).

8. The method of claim 7, wherein DMOG is present in the medium in a concentration ranging from 8 to 50 µg/mL.

9. The method of claim 8, wherein DMOG is present in the medium in a concentration of 32 µg/mL.

10. The method according to any of the preceding claims, wherein said leukocytes comprise human granulocytes.

11. The method according to claim 10, wherein said human granulocytes comprise polymorphonuclear neutrophils.

12. The method of claim 11, wherein said polymorphonuclear neutrophils are purified.

13. The method of claim 12, wherein the polymorphonuclear neutrophils have been purified from citrated venous blood.

14. The method according to any of the preceding claims, wherein more than 70% of the polymorphonuclear neutrophils remain viable after 20 hours storage.

15. The method according to any of the preceding claims, wherein more than 45% of the polymorphonuclear neutrophils remain viable after 48 hours storage.

16. The method according to any of the preceding claims, wherein the viable polymorphonuclear neutrophils remain functional.

17. The method according to claims 1 to 9, wherein said leukocytes comprise human peripheral blood mononuclear cells (PBMCs).

18. An oxygen-tight container containing live leukocytes in a medium with P(O₂) ≤ 10 mM Hg and from 3 to 10 mM of glucose.

19. A device for transporting leukocytes, comprising an oxygen-tight container containing a culture medium as recited in any of claims 1 to 9, and means appropriate for introducing cells into the culture medium.

20. The device of claim 19, comprising polymorphonuclear neutrophils.

21. A culture medium for neutrophils, **characterized in that** it comprises between 3 and 6 mM of glucose and between 25 and 40 µg/mL of DMOG.

22. A method for transfecting polymorphonuclear neutrophils, comprising a step of pre-conditioning said polymorphonuclear neutrophils by transferring them into a medium as recited in any of claims 1-9 and incubating them in hypoxic or anoxic conditions, followed by a transfection step.

23. A population of polymorphonuclear neutrophils which has been transfected according to the method of claim 22, **characterized in that** at least 30% of the cells have been effectively transfected.


SEQUENCE LISTING

<110>  INSTITUT PASTEUR

<120>  METHOD AND DEVICE FOR CONSERVING VIABLE AND FUNCTIONAL HUMAN
       POLYMORPHONUCLEAR NEUTROPHILS

<130>  VMAahF226/201EP

<160>  4

<170>  PatentIn version 3.5

<210>  1
<211>  20
<212>  DNA
<213>  Artificial Sequence

<220>
<223>  Primer

<400>  1
gccttcctga tttctgcagc                                                   20


<210>  2
<211>  19
<212>  DNA
<213>  Artificial Sequence

<220>
<223>  Primer

<400>  2
tgcacccagt tttccttgg                                                    19


<210>  3
<211>  17
<212>  DNA
<213>  Artificial Sequence

<220>
<223>  Primer

<400>  3
tcgctctctg ctcctcc                                                      17


<210>  4
<211>  20
<212>  DNA
<213>  Artificial Sequence

<220>
<223>  Primer

<400>  4
ttaaaagcag ccctggtgac                                                   20